(19) Europäisches Patentamt
European Patent Office
Office européen des brevets

(11) Numéro de publication: 0 135 400
B1

# (12) FASCICULE DE BREVET EUROPEEN

(45) Date de publication du fascicule du brevet: **01.06.88**

(51) Int. Cl.⁴: **C 07 J 41/00**, C 07 B 59/00, A 61 K 49/02, G 01 N 33/74

(21) Numéro de dépôt: **84401203.9**

(22) Date de dépôt: **13.06.84**

(54) **Dérivés estradiéniques radioactifs marqués au tritium, leur procédé et leurs intermédiaires de préparation et leur application pour l'étude et le dosage radioimmunologique de stéroides dans les fluides biologiques.**

(30) Priorité: **14.06.83 FR 8309812**

(43) Date de publication de la demande: **27.03.85 Bulletin 85/13**

(45) Mention de la délivrance du brevet: **01.06.88 Bulletin 88/22**

(84) Etats contractants désignés: **AT BE CH DE GB IT LI LU NL SE**

(56) Documents cités:
**EP - A - 0 007 823**
**FR - A - 2 414 515**
**FR - A - 2 497 807**

(73) Titulaire: **ROUSSEL-UCLAF, 35, boulevard des Invalides, F-75007 Paris (FR)**

(72) Inventeur: **Jouquey, Alain, 137, rue des Pyrénées, F-75020-Paris (FR)**
Inventeur: **Veltz, Jean-Noel, 75, rue de Strasbourg, F-93200-Saint-Denis (FR)**
Inventeur: **Salmon, Jean, 8, rue du Général Castelnau, F-75015-Paris (FR)**
Inventeur: **Mouren, Michel, 184, avenue de Choisy, F-75013-Paris (FR)**

(74) Mandataire: **Bourgouin, André et al, Département des Brevets ROUSSEL UCLAF B.P. no 9, F-93230 Romainville (FR)**

**EP 0 135 400 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés estradiéniques radioactifs marqués au tritium, leur procédé et leurs intermédiaires de préparation et leur application pour l'étude et le dosage de stéroïdes dans les fluides biologiques.

Le brevet français 2 497 807 décrit des composés 19-nor stéroïdes ou 19-nor D-homostéroïdes substitués en position 11β par un radical organique, comportant au moins un atome d'azote de silicium ou de phosphore mais ce brevet ne décrit aucun produit tritié.

Le brevet français 2 414 515 décrit des dérivés comprenant le squelette estra-5(10),9(11)-diène tritiés en position 6 et 7 mais qui ne comportent pas de substituant en position 11.

La demande de brevet européen 0 007 823 décrit des stéroïdes 3,20-dioxo 4,9-diène 21-hydroxylés pouvant être tritiés en position 6 et 7 mais qui ne sont pas substitués en position 11.

La présente invention a plus particulièrement pour objet des dérivés estradiéniques radioactifs marqués au tritium de formule générale (I):

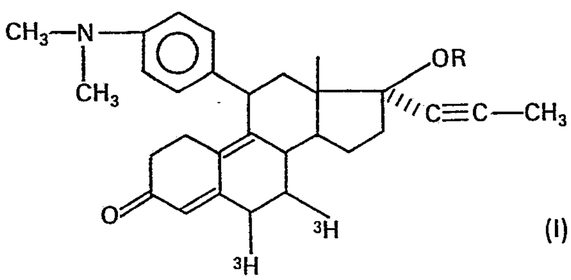

(I)

dans laquelle le symbole $^3H$ représente le tritium et R représente un atome d'hydrogène, un radical alcoyle ayant de 1 à 6 atomes de carbone ou un radical acyle ayant de 1 à 10 atomes de carbone.

Parmi les valeurs de R, on peut citer:

a) les radicaux méthyle, éthyle, propyle, isopropyle, butyle, sec-butyle, tert-butyle, pentyle, iso-pentyle, sec-pentyle, tert-pentyle, néo-pentyle, hexyle, iso-hexyle, sec-hexyle, tert-hexyle;

b) les radicaux formyle, acétyle, propionyle, butyryle, isobutyryle, valéryle, caproyle, benzoyle, caprylyle, méthoxy-carbonyle ou éthoxy. Parmi les produits de formule générale (I), on citera notamment:

- la 17β-hydroxy-11β-(4-diméthylamino phényl)-17α-(prop-l-ynyl)estra-4,9-dièn-3-one (6,7-$^3H$), qui sera désignée dans ce qui suit comme produit A.

Les produits de formule générale (I) permettent notamment l'étude et le dosage radioimmunologique de la 17β -hydroxy-11β -(4-diméthylaminophényl)-17α-(prop-1-ynyl)estra-4,9-dièn-3-one non radioactive, qui sera désignée dans ce qui suit comme produit B.

L'invention a également pour objet un procédé de préparation des produits répondant à la formule générale (I) ci-dessus.

Ce procédé est caractérisé en ce que:

- on bloque la fonction cétone en 3 du produit de formule (II) dans laquelle le symbole $^3H$ représente le tritium:

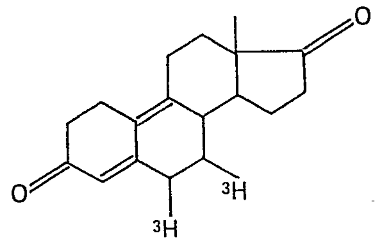

(II)

au moyen d'un agent de cétalisation,

- soumet le produit de formule générale (III) résultant:

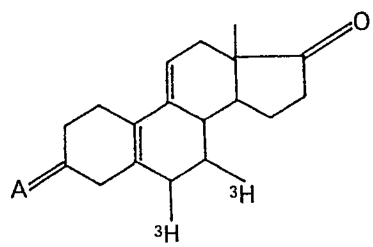

(III)

formule (III) dans laquelle A représente un groupement oxo bloqué sous forme de cétal ouvert ou cyclique, à l'action d'un agent de propynylation, obtient le produit de formule générale (IV):

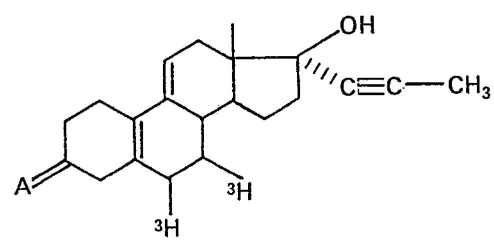

(IV)

- que l'on soumet à l'action d'un agent d'époxydation pour obtenir le produit de formule générale (V):

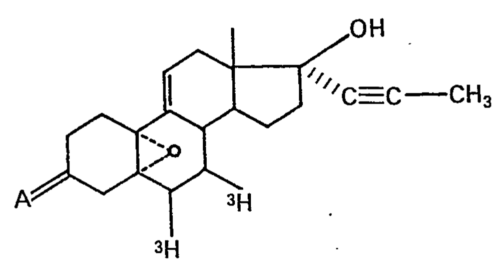

(V)

- sur lequel on fait agir un halogénure de 4-diméthylamino phényl magnésium,

- puis soumet le produit de formule générale (VI) résultant:

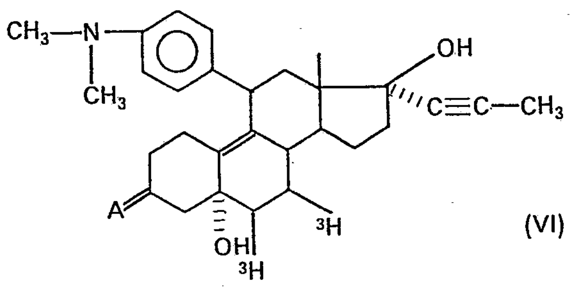

(VI)

à l'hydrolyse et obtient le produit de formule générale (I) cherché, dans laquelle R représente un atome d'hydrogène dont, le cas échéant, on éthérifie ou estérifie la fonction hydroxyle en 17.

Dans une mise en œuvre préférentielle, ce procédé est encore caractérisé en ce que:

- l'agent de cétalisation au moyen duquel on bloque la fonction en 3 est un alcanol ayant de 1 à 4 atomes de carbone ou un glycol ayant 2 atomes de carbone substitués ou non par un ou deux groupements alcoyle, tel que méthyle, et on opère sous atmosphère inerte,
- l'agent de propynylation que l'on fait agir sur le produit de formule générale (III) est le bromure de propynylmagnésium et on opère sous atmosphère inerte,
- l'agent d'epoxydation que l'on fait agir sur le produit de formule générale (IV) est un peracide tel que l'acide m-chloro perbenzoïque ou l'eau oxygénée en présence d'hydrate d'hexafluoroacétone et on opère sous atmosphère inerte,
- l'halogénure de 4-diméthylaminophényl magnésium que l'on fait agir sur le produit de formule générale (V) est le bromure et on opère sous atmosphère inerte,
- on soumet le produit de formule générale (VI) à l'hydrolyse acide au moyen d'acide chlorhydrique et on opère sous atmosphère inerte.

L'éthérification ou l'estérification de la fonction hydroxyle en 17 du produit de formule générale (I), dans laquelle R représente un atome d'hydrogène, peut être effectuée selon les méthodes habituelles.

L'éthérification peut être effectuée par exemple au moyen d'un agent d'éthérification tel qu'un halogénure d'alcoyle ou d'aryle, ou un sulfate de dialcoyle.

L'estérification peut être effectuée par exemple au moyen d'un agent d'estérification tel qu'un dérivé fonctionnel d'acide, comme un chlorure d'acide ou un anhydride.

Selon une variante du procédé de l'invention, également objet de l'invention:

- on bloque la fonction cétone en 3 du produit de formule (II) sous forme d'un cétal ouvert, au moyen d'un agent de cétalisation de formule générale $R_1O4$, dans laquelle $R_1$ représente un radical alcoyle ayant de 1 à 4 atomes de carbone,
- soumet le produit de formule générale (VII) résultant:

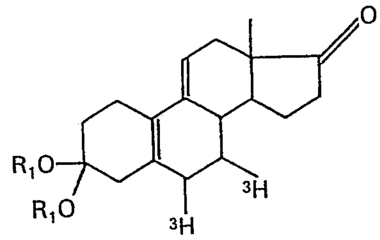

(VII)

à l'action d'un agent de propynylation, obtient le produit de formule générale (VIII):

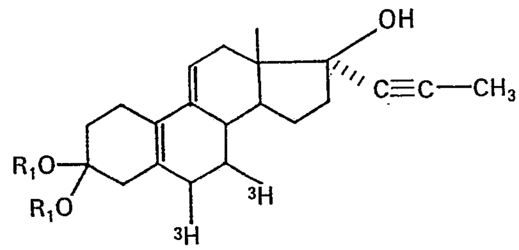

(VIII)

- effectue la transcétalisation de la fonction cétal au moyen d'un glycol de formule $R_2CHOH$-$CHOHR_3$ en milieu acide, pour obtenir le produit de formule générale (IX):

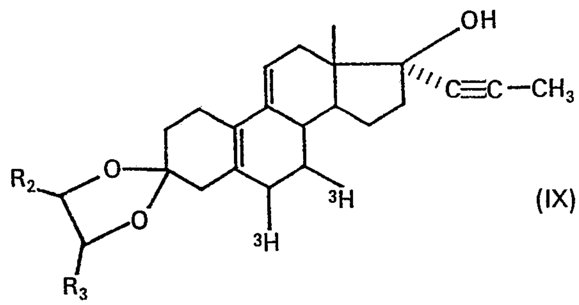

(IX)

dans laquelle $R_2$ et $R_3$, identiques ou différents, représentent un atome d'hydrogène ou un radical alcoyle tel que méthyle, que l'on soumet selon le procédé indiqué ci-dessus, à l'action d'un agent d'époxydation, puis fait agir sur le produit résultant un halogénure de 4-diméthylaminophényl magnésium et finalement soumet le produit résultant à l'hydrolyse pour obtenir le produit de formule générale (I) cherché.

Dans une mise en œuvre préférentielle, la variante du procédé de l'invention peut encore être caractérisée en ce que:

- l'agent de cétalisation au moyen duquel on bloque la fonction cétone en 3 du produit de formule (II) est le méthanol et on opère sous atmosphère inerte,
- l'agent de propynylation que l'on fait agir sur le produit de formule générale (VII) est le bromure de propynylmagnésium et on opère sous atmosphère inerte,
- le glycol au moyen duquel on effectue la transcétalisation de la fonction cétone du produit de formule générale (VIII) est l'éthylène glycol et on opère sous atmosphère inerte.

Le procédé de l'invention est encore illustré en ce que:

- on choisit favorablement comme atmosphère

inerte, sous laquelle il est exécuté, l'atmosphère d'argon,
- on fait agir un halogénure de 4-diméthylamino phényl magnésium sur un produit de formule générale (V) en présence d'un sel cuivreux,
- on fait agir un alcanol comme agent de cétalisation au moyen duquel on bloque la fonction en 3, en milieu acide, tel que le mélange acide paratoluène sulfonique-acide chlorhydrique ou acide chlorhydrique-pyridine et éventuellement en présence d'un déshydratant,
- on fait agir un glycol comme agent de cétalisation au moyen duquel on bloque la fonction en 3, en présence d'un déshydratant,
- on fait agir l'agent de propynylation sur un produit de formule générale (III) en opérant dans le tétrahydrofurane,
- l'agent d'époxydation que l'on fait agir sur un produit de formule génerale (IV) est l'acide m-chloro perbenzoïque,
- on fait agir le bromure de 4-diméthylamino phényl magnésium sur un produit de formule générale (V) en opérant dans le tétrahydrofuranne et en présence d'un sel cuivreux,
- la transcétalisation de la fonction cétal d'un produit de formule générale (VIII) au moyen d'un glycol est effectuée en milieu acide par exemple au sein du chlorhydrate de pyridine.

L'invention a également pour objet l'application des produits de formule générale (I) et notamment du produit A à l'étude et au dosage radioimmunologique du produit B et de ses métabolites dans les fluides biologiques chez l'homme ou chez l'animal.

L'invention a également pour objet un moyen pour l'étude et le dosage radioimmunologique du produit B et de ses métabolites, comportant un produit de formule générale (I) et notamment le produit A.

Le produit A, après son administration peut être suivi dans son évolution et dans son comportement au niveau des fluides biologiques lors des études pharmacologiques et cliniques.

Lors de ces études, le produit A permet notamment un dosage specifique aisé de quantités de l'ordre de quelques dizaines de picogrammes par ml de fluide biologique, sans que l'on soit obligé de recourir à des méthodes d'isolement et de purification par chromatographie, avant de procéder au dosage proprement dit.

Pour étudier et doser par des méthodes radioimmunologiques la 17β-hydroxy-11β-(4-diméthylamino phényl)-17α -(prop-1-ynyl)estra-4,9-dièn-3-one non radioactive (produit B), on prépare en premier lieu à partir de cette dernière, selon des méthodes connues en soi, des antigènes, en la conjugant avec l'albumine sérique bovine (BSA) ou avec l'albumine sérique humaine (HSA).

Les antigènes résultants peuvent servir au développement d'anticorps lorsqu'il sont injectés chez l'animal, en présence d'un adjuvant. Ils permettent ainsi d'obtenir de sérums contenant des anticorps.

Ces anticorps servent ensuite de récepteurs de produits radioactifs et/ou de produits non-radioactifs, à savoir de récepteurs des produits de formule générale (I) et notamment de la 17β-hydroxy-11β-(4-diméthylaminophényl)-17 α -(prop-1-ynyl) estra-dièn-3-one (6,7-$^3$H) (produit A) ainsi que de la 17β-hydroxy-11β-(4-diméthylamino phényl)-17α-(prop-1-ynyl-estra-4,9-dièn-3-one non radioactive (produit B).

La présence de ces anticorps est mise en évidence au moyen d'un produit de formule générale (I) et notamment au moyen du produit A.

On procède ensuite au dosage du produit B selon des méthodes radioimmunologiques conventionnelles, telles que celles décrites par:
- S.A. Bergson et R.S. Yalow, Hormone 4, p. 557 (1964),
- C.E. Abraham, J. of Chem. Endocrinal. Metab. 29, p. 866 (1969).

Les produits de formule générale (I) et notamment le produit A peuvent en outre être utilisés pour le dosage et la localisation des récepteurs de stéroïdes, tels que les récepteurs de glucocorticocoïdes, de progestagènes et d'androgènes dans les cellules des tissus cibles.

Le dosage et la localisation de ces récepteurs peuvent être effectués selon des méthodes habituelles, telles que celles décrites par:
- Raynaud J. P., Philibert D. et al., J. of Steroid Biochem., 6 (1975), p. 615-622,
- Moguilewsky et al., J. of Steroid Biochem. 12 (1980), p. 309-314.

L'invention a également pour objet à titre de produits industriels nouveaux et notamment à titre de produits intermédiaires nécessaires à l'exécution du procédé de l'invention:
- le 3,3-diméthoxy-17 β-hydroxy-17α -(prop-1-ynyl)-estra-5(10), 9(11)-diène(6,7-$^3$H),
- le 3,3-éthylène dioxy-17β -hydroxy-17α -(prop-1-ynyl)-estra-5(10), 9(11)-diène (6,7-$^3$H),
- le 3,3-éthylène dioxy-5α, 10α-époxy-17β-hydroxy-17α-(prop-1-ynyl)-estra 9(11)-ène (6,7-$^3$H),
- le 3,3-éthylène dioxy-5α, 17β-dihydroxy-11β-(4-diméthylamino phényl)-17α-(prop-1-ynyl)-estra-9(11)-ène (6,7-$^3$H).

L'exemple suivant illustre l'invention sans toutefois la limiter.

Exemple

17β-hydroxy-11β-(4-diméthylamino phényl)-17α-(prop-1-ynyl)-estra-4,9-dièn-3-one (6,7-$^3$H).

Stade A: 3,3-diméthoxy-17-oxo-estra-5(10), 9(11)-diène (6,7-$^3$H).

On introduit sous atmosphère inerte, 8 Ci soit 40 mg de 3,17-dioxo estra-5(10), 9(11)-diène (6,7-$^3$H) dans 0,5 ml de diméthylcétal de l'acétone, puis ajoute 50 µl de méthanol contenant 0,5% de chlorure d'acétyle, laisse au repos le mélange réactionnel pendant 15 minutes environ, puis additionne de 20 µl de triéthylamine et concentre sous vide. On obtient le produit recherché que l'on utilise tel que pour le stade suivant de la synthèse.

La chromatographie sur couche mince de silice avec élution par le système cyclohexane/acétate d'éthyle (75:25) donne Rf=0,25.

Le produit de départ a été décrit dans la publication J. Salmon: «Synthèse et application de la 17α-méthyl triénolone tritiée» (Symposium sur le progrès des techniques nucléaires en pharmacodynamie - Saclay 11-13 Mars 1970, Ed; Masson; G. Valette et Y. Cohen, p. 237-248).

Stade B: 3,3-diméthoxy-17β-hydroxy-17α-(prop-1-ynyl)estra-5(10), 9(11)-diène(6,7-$^3$H).

On reprend sous atmosphère inerte le produit obtenu au stade précédent par 0,5 ml de tétrahydrofuranne, ajoute 0,5 ml d'une suspension de bromure de propynyl magnésium 1M dans le tétrahydrofuranne et agite le mélange réactionnel à température ambiante pendant 1 heure environ.

On ajoute ensuite 1 ml d'une solution aqueuse de chlorure d'ammonium à 10%, extrait à l'acétate d'éthyle, lave à l'eau, amène à sec sous pression réduite et obtient le produit cherché que l'on utilise tel que pour le stade suivant de la synthèse.

La chromatographie sur couche mince de silice avec élution par le système cyclohexane/acétate d'éthyle (75/25) donne Rf = 0,22.

Stade C: 3,3-éthylène dioxy-17β-hydroxy-17α-(prop-1-ynyl)estra-5(10), 9(11)-diène (6,7-$^3$H).

On reprend sous atmosphère inerte le produit obtenu au stade précédent par 1 ml de glycol anhydre, ajoute 0,5 mg de chlorhydrate de pyridine, chauffe 30 mn environ à 60°C, ajoute 2 ml d'eau, extrait à l'acétate d'éthyle, lave à l'eau, sèche, amène à sec et obtient le produit cherché.

La chromatographie sur couche mince de silice avec élution par le système cyclohexane/acétate d'éthyle (75/25) donne Rf = 0,2.

Le produit peut être purifié par chromatographie liquide haute performance sur silice, avec l'élution par le système cyclohexane/acétate d'éthyle à 0,06% d'eau/triéthylamine (75:24:1).

On obtient 2,44 Ci soit 15 mg de produit cherché.

Stade D: 3,3-éthylène dioxy-5α, 10α-époxy-17β-hydroxy-17α-(prop-1-ynyl)estra 9(11)-ène (6,7-$^3$H).

On dissout sous atmosphère inerte 15 mg de produit obtenu au stade précédent dans 0,5 ml de chlorure de méthylène, ajoute 10 mg d'oxyde de magnésium, refroidit à -10°C, puis ajoute sous agitation 12 mg d'acide m-chloroperbenzoïque, maintient l'agitation pendant 1 heure environ à -10°C, additionne ensuite 1 ml d'une solution aqueuse de thiosulfate de sodium à 0,2N, extrait à l'acétate d'éthyle, lave à l'eau et amène à sec sous pression réduite.

On obtient le produit cherché que l'on utilise tel que pour le stade suivant de la synthèse.

La chromatographie sur couche mince de silice avec élution par le système cyclohexane/acétate d'éthyle (75/25) donne Rf=0,1.

Stade E: 3,3-éthylène dioxy 5α, 17β-dihydroxy-11β-(4-diméthylamino phényl-17α-(prop-1-ynyl)estra-9-ène (6,7-$^3$H).

On dissout sous atmosphère inerte le produit obtenu au stade précédent dans 0,5 ml de tétrahydrouranne, ajoute 20 mg d'iodure cuivreux, agite pendant 20 minutes environ puis ajoute 0,5 ml d'une solution de bromure de 4-diméthylamino phényl magnésium 1M dans le tétrahydrofuranne. On maintient le mélange réactionnel sous agitation, à température ambiante, pendant 1 heure environ, puis hydrolyse par 0,5 ml d'une solution aqueuse de chlorure d'ammonium à 10% et 0,2 ml d'une solution aqueuse 0,2M de thiosulfate de sodium. On extrait à l'acétate d'éthyle, lave à l'eau, sèche et obtient le produit cherché.

La chromatographie sur couche mince de silice avec élution par le système cyclohexane/acétate d'éthyle (50:50) donne Rf=0,22.

Le produit peut être purifié par chromatographie liquide haute performance sur silice, avec élution par le système cyclohexane/acétate d'éthyle à 0,06% d'eau (70:30) renfermant 0,05% de triéthylamine. On obtient 1,08 Ci soit 10 mg de produit cherché.

Stade F: 17β-hydroxy-11β-(4-diméthylamino phényl)-17α-(prop-1-ynyl)-estra-4,9-dièn-3-one (6,7-$^3$H).

On dissout sous atmosphère inerte 10 mg de produit obtenu au stade précédent dans 0,5 ml de méthanol, ajoute sous agitation 20 µl d'acide chlorhydrique concentré, maintient l'agitation pendant une demi-heure à température ambiante, ajoute 1 ml d'acétate d'éthyle puis 1 ml de soude N, sépare la phase organique, lave à l'eau, sèche et amène à sec sous pression réduite et obtient le produit cherché brut.

La chromatographie sur couche mince de silice avec élution par le système cyclohexane/acétate d'éthyle (50:50) donne Rf=0,25.

Ce produit peut être purifié par chromatographie liquide haute performance sur silice avec élution par le système cyclohexane/acétate d'éthyle à 0,06% d'eau (70:30). On obtient 6,3 mg de produit cherché, d'une activité spécifique de 37,5 Ci $mol^{-1}$.

**Revendications pour les Etats contractants BE, CH, DE, GB, IT, LI, LU, NL, SE**

1. Dérivés estradiéniqeues radioactifs marqués au tritium de formule générale (I):

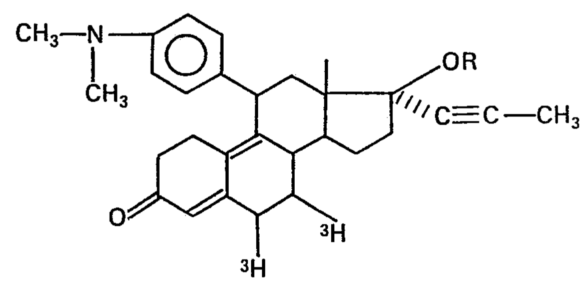

dans laquelle $^3H$ représente le tritium et R représente un atome d'hydrogène, un radical alcoyle ayant de 1 à 6 atomes de carbone ou un radical acyle ayant de 1 à 10 atomes de carbone.

2. La 17β-hydroxy-11β-(4-diméthylamino phényl)-17α-(prop-1-ynyl)estra-4,9-dièn-3-one (6,7-$^3H$).

3. Procédé de préparation des produits définis aux revendications 1 et 2 caractérisé en ce que:

- on bloque la fonction cétone en 3 du produit de formule (II) dans laquelle le symbole $^3H$ représente le tritium:

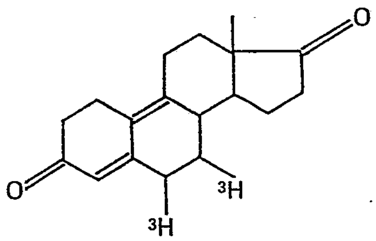

(II)

au moyen d'un agent de cétalisation,

- soumet le produit de formule générale (III) résultant:

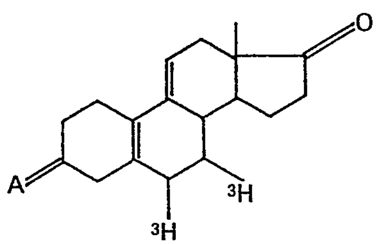

(III)

formule (III) dans laquelle A représente un groupement oxo bloqué sous forme de cétal ouvert ou cyclique, à l'action d'un agent de propynylation, obtient le produit de formule générale (IV):

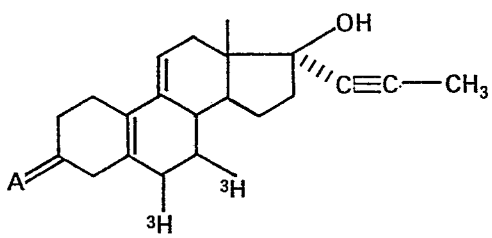

(IV)

- que l'on soumet à l'action d'un agent d'époxydation pour obtenir le produit de formule générale (V):

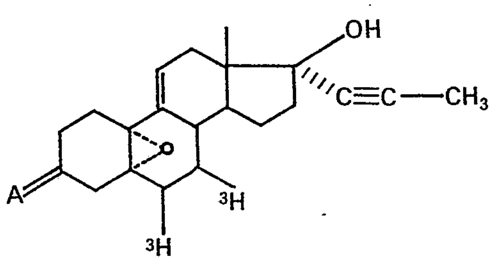

(V)

- sur lequel on fait agir un halogénure de 4-diméthylamino phényl magnésium,

- puis soumet le produit de formule générale (VI) résultant:

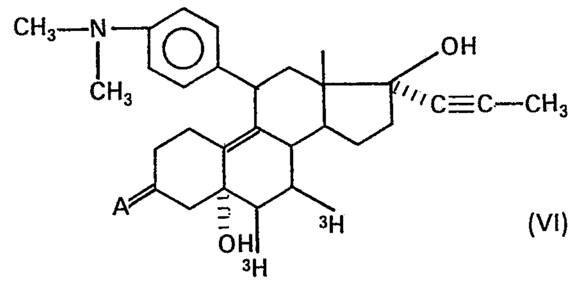

(VI)

à l'hydrolyse et obtient le produit de formule générale (I) cherché, dans laquelle R représente un atome d'hydrogène dont, le cas échéant, on éthérifie ou estérifie la fonction hydroxyle en 17.

4. Procédé suivant la revendication 3, caractérisé en ce que:

- l'agent de cétalisation au moyen duquel on bloque la fonction en 3 est un alcanol ayant de 1 à 4 atomes de carbone ou un glycol ayant 2 atomes de carbone substitués ou non par un ou deux groupements alcoyle, tel que méthyle, et on opère sous atmosphère inerte,

- l'agent de propynylation que l'on fait agir sur le produit de formule générale (III) est le bromure de propynylmagnésium et on opère sous atmosphère inerte,

- l'agent d'époxydation que l'on fait agir sur le produit de formule générale (IV) est un peracide tel que l'acide m-chloro perbenzoïque ou l'eau oxygénée en présence d'hydrate d'hexafluoroacétone et on opère sous atmosphère inerte,

- l'halogénure de 4-diméthylaminophényl magnésium que l'on fait agir sur le produit de formule générale (V) est le bromure et on opère sous atmosphère inerte,

- on soumet le produit de formule générale (VI) à l'hydrolyse acide au moyen d'acide chlorhydrique et on opère sous atmosphère inerte.

5. Variante du procédé suivant la revendication 3 caractérisé en ce que:

- on bloque la fonction cétone en 3 du produit de formule (II) sous forme d'un cétal ouvert, au moyen d'un agent de cétalisation de formule générale $R_1OH$, dans laquelle $R_1$ représente un radical alcoyle ayant de 1 à 4 atomes de carbone,

- soumet le produit de formule générale (VII) résultant:

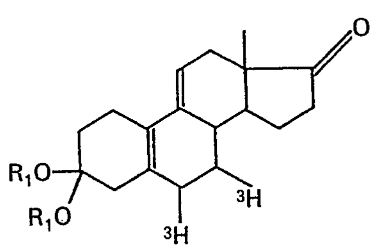

(VII)

à l'action d'un agent de propynylation, obtient le produit de formule générale (VIII):

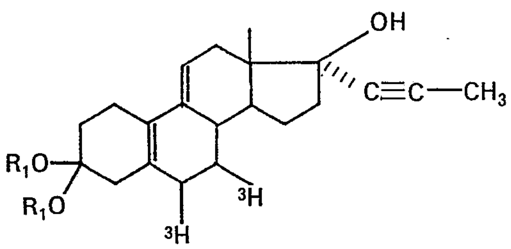

(VIII)

- effectue la transcétalisation de la fonction cétal au moyen d'un glycol de formule $R_2CHOH$-$CHOHR_3$ en milieu acide, pour obtenir le produit de formule générale (IX):

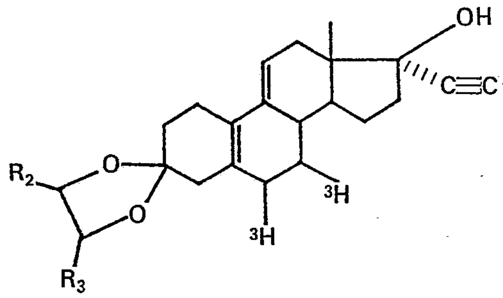

(IX)

dans laquelle $R_2$ et $R_3$, identiques ou différents, représentent un atome d'hydrogène ou un radical alcoyle tel que méthyle, que l'on soumet selon le procédé indiquée ci-dessus, à l'action d'un agent d'époxydation, puis fait agir sur le produit résultant un halogénure de 4-diméthylaminophényl magnésium et finalement soumet le produit résultant à l'hydrolyse pour obtenir le produit de formule générale (I) cherché.

6. Procédé suivant la revendication 5 caractérisé en ce que:
- l'agent de cétalisation au moyen duquel on bloque la fonction cétone en 3 du produit de formule (II) est le méthanol et on opère sous atmosphère inerte,
- l'agent de propynylation que l'on fait agir sur le produit de formule générale (VII) est le bromure de propynylmagnésium et on opère sous atmosphère inerte,
- le glycol au moyen duquel on effectue la transcétalisation de la fonction cétone du produit de formule générale (VIII) est l'éthylène glycol et on opère sous atmosphère inerte.

7. Application des produits tels que définis par la formule générale (I) de la revendication 1 et notamment de la 17β-hydroxy11β-(4-diméthylamino-phényl)-17α-(prop-1-ynyl) estra 4,9-dièn-3-one(6,7-$^3$H) pour l'étude et le dosage radioimmunologique de la 17β-hydroxy-11β-(4-diméthylaminophényl)-17α-(prop-1-ynyl) estra 4,9-dièn-3-one non radioactive et de ses métabolites dans les fluides biologiques chez l'homme ou l'animal.

8. Les produits de formule (I), tels que définis à la revendication 1 ou 2, pour leur utilisation, chez l'homme ou l'animal, pour l'étude et le dosage radioimmunologique de la 17β-hydroxy 11β-(4-diméthylaminophényl) 17α-(prop-1-ynyl) estra 4,9-dièn-3-one non radioactive et de ses métabolites, dans les fluides biologiques.

9. Moyen pour l'étude et le dosage radioimmunologique de la 17β-hydroxy 11β-(4-diméthylaminophényl) 17α-(prop-1-ynyl) estra 4,9-dièn-3-one non radioactive et de ses métabolites dans les fluides biologiques chez l'homme ou l'animal, comportant un produit de formule générale (I) de la revendication 1 et notamment la 17β-hydroxy 11β-(4-diméthylaminophènyl) 17α-(prop-1-ynyl) estra 4,9-dièn-3-one (6,7-$^3$H).

10. A titre de produits industriels nouveaux:
- le 3,3-diméthoxy-17β-hydroxy-17α-(prop-1-ynyl)-estra-5(10), 9(11)-diène (6,7-$^3$H),
- le 3,3-éthylène dioxy-17β -hydroxy-17α -(prop-1-ynyl)-estra-5(10), 9(11)-diène(6,7-$^3$H),
- le 3,3-éthylène dioxy-5α, 10α-époxy-17β-hydroxy-17α-(prop-1-ynyl)-estra 9(11)-ène (6,7-$^3$H),
- le 3,3-éthylène dioxy-5α, 17β -dihydroxy-11β-(4-diméthylamino-phényl)-17α-(prop-1-ynyl)-estra-9(11)-ène (6,7-$^3$H).

**Revendications pour l'Etat contractant: AT**

1. Procédé de préparation des dérivés estradiéniques radioactifs marqués au tritium de formule générale (I):

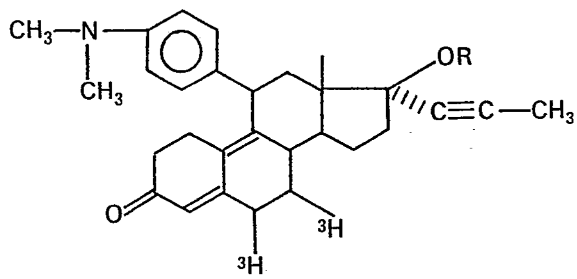

dans laquelle $^3$H représente le tritium et R représente un atome d'hydrogène, un radical alcoyle ayant de 1 à 6 atomes de carbone ou un radical acyle ayant de 1 à 10 atomes de carbone, caractérisé en ce que:
- on bloque la fonction cétone en 3 du produit de formule (II) dans laquelle le symbole $^3$H représente le tritium:

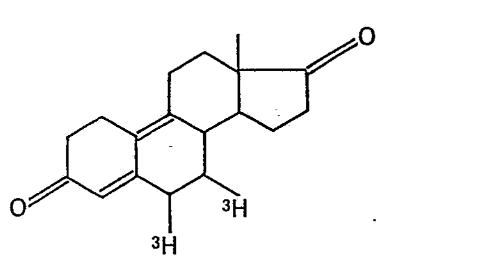

(II)

au moyen d'un agent de cétalisation,
- soumet le produit de formule générale (III) résultant:

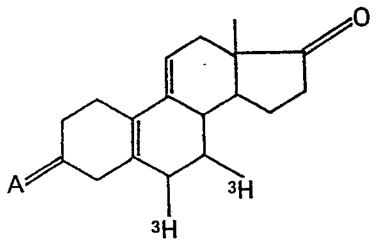

(III)

formule (III) dans laquelle A représente un groupement oxo bloqué sous forme de cétal ouvert ou cyclique, à l'action d'un agent de propynylation, obtient le produit de formule générale (IV):

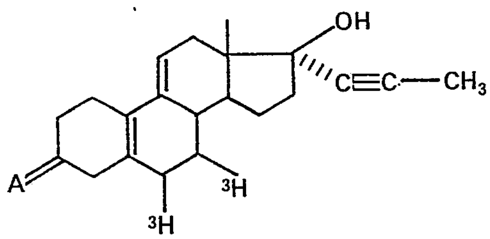

(IV)

- que l'on soumet à l'action d'un agent d'époxydation pour obtenir le produit de formule générale (V):

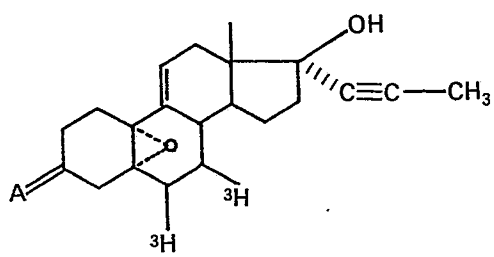

(V)

- sur lequel on fait agir un halogénure de 4-diméthylamino phényl magnésium,
- puis soumet le produit de formule générale (VI) résultant:

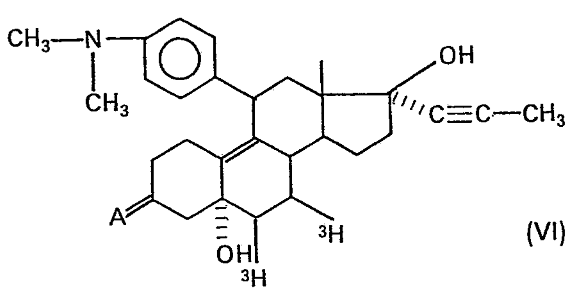

(VI)

à l'hydrolyse et obtient le produit de formule générale (I) cherché, dans laquelle R représente un atome d'hydrogène dont, le cas échéant, on éthérifie ou estérifie la fonction hydroxyle en 17.

2. Procédé suivant la revendication 1, caractérisé en ce que:
- l'agent de cétalisation au moyen duquel on bloque la fonction en 3 est un alcanol ayant de 1 à 4 atomes de carbone ou un glycol ayant 2 atomes de carbone substitués ou non par un ou deux groupements alcoyle, tel que méthyle, et on opère sous atmosphère inerte,
- l'agent de propynylation que l'on fait agir sur le produit de formule générale (III) est le bromure de propynylmagnésium et on opère sous atmosphère inerte,
- l'agent d'époxydation que l'on fait agir sur le produit de formule générale (IV) est un peracide tel que l'acide m-chloro perbenzoïque ou l'eau oxygénée en présence d'hydrate d'hexafluoroacétone et on opère sous atmosphère inerte,
- l'halogénure de 4-diméthylaminophényl magnésium que l'on fait agir sur le produit de formule générale (V) est le bromure et on opère sous atmosphère inerte,
- on soumet le produit de formule générale (VI) à l'hydrolyse acide au moyen d'acide chlorhydrique et on opère sous atmosphère inerte.

3. Variante du procédé suivant la revendication 1 caractérisé en ce que:
- on bloque la fonction cétone en 3 du produit de formule (II) sous forme d'un cétal ouvert, au moyen d'un agent de cétalisation de formule générale $R_1OH$, dans laquelle $R_1$ représente un radical alcoyle ayant de 1 à 4 atomes de carbone,
- soumet le produit de formule générale (VII) résultant:

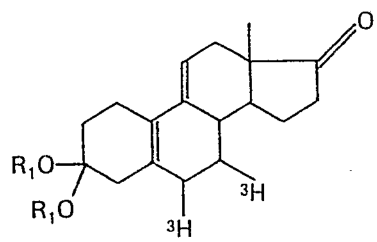

(VII)

à l'action d'un agent de propynylation, obtient le produit de formule générale (VIII):

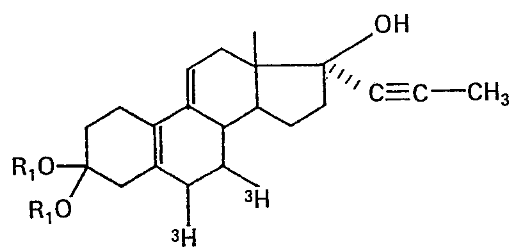

(VIII)

- effectue la transcétalisation de la fonction cétal au moyen d'un glycol de formule $R_2CHOH-CHOHR_3$ en milieu acide, pour obtenir le produit de formule générale (IX):

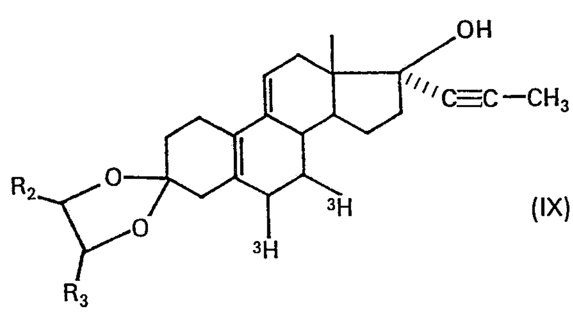

(IX)

dans laquelle $R_2$ et $R_3$, identiques ou différents, représentent un atome d'hydrogène ou un radical alcoyle tel que méthyle, que l'on soumet selon le procédé indiquée ci-dessus, à l'action d'un agent d'époxydation, puis fait agir sur le produit résultant lun halogénure de 4-diméthylaminophényl magnésium et finalement soumet le produit résultant à l'hydrolyse pour obtenir le produit de formule générale (I) herché.

4. Procédé suivant la revendication 3 caractérisé en ce que:

- l'agent de cétalisation au moyen duquel on bloque la fonction cétonne en 3 du produit de formule (II) est le méthanol et on opère sous atmosphère inerte,
- l'agent de propynylation que l'on fait agir sur le produit de formule générale (VII) est le bromure de propnylmagnésium et on opère sous atmosphère inerte,
- le glycol au moyen duquel on effectue la transcétalisation de la fonction cétone du produit de formule générale (VIII) est l'éthylène glycol et on opère sous atmosphère inerte.

5. Application des produits tels que définis par la formule générale (I) de la revendication 1 et notamment de la 17β-hydroxy-11β-(4-diméthylaminophényl)-17α-(prop-1-ynyl) estra 4,9-dièn-3-one(6,7-$^3H$) pour l'étude et le dosage radioimmunologique de la 17β-hydroxy-11β-(4-diméthylaminophényl)-17α-(prop-1-ynyl) estra 4,9-dièn-3-one non radioactive etde ses metabolites dans les fluides biologiques chez l'homme ou l'animal.

6. Moyen pour l'étude et le dosage radioimmunologique de la 17β-hydroxy 11β-(4-diméthylaminophényl) 17α-(prop-1-ynyl) estra 4,9-dièn-3-one non radioactive et de ses métabolites dans les fluides biologiques chez l'homme ou l'animal, comportant un produit de formule générale (I) de la revendication 1 et notamment la 17β-hydroxy 11β-(4-diméthylaminophényl)17α-(prop-1-ynyl) estra 4,9-dièn-3-one (6,7-$^3H$).

**Patentansprüche für die Vertragsstaaten: BE, CH, DE, GB, IT, LI, LU, NL, SE**

1. Tritium-markierte, radioaktive Östradien-Derivate der allgemeinen Formel (I)

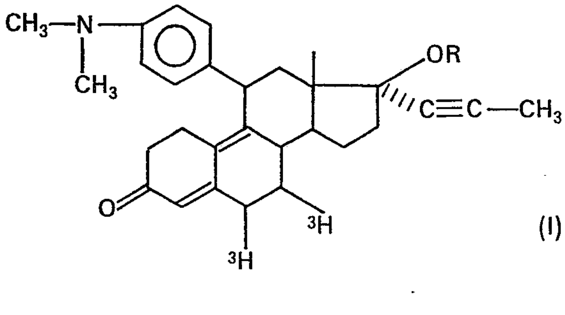

(I)

worin $^3H$ für das Tritium steht und R ein Wasserstoffatom, einen Alkylrest mit 1 bis 6 Kohlenstoffatomen oder einen Acylrest mit 1 bis 10 Kohlenstoffatomen darstellt.

2. 17β-Hydroxy-11β-(4-dimethylaminophenyl)-17α-(prop-1-inyl)-östra-4,9-dien-3-on (6,7-$^3H$).

3. Verfahren zur Herstellung der in den Ansprüchen 1 und 2 definierten Produkte, dadurch gekennzeichnet, dass man die Ketonfunktion in 3-Stellung des Produkts der Formel (II), worin das Symbol $^3H$ für Tritium steht,

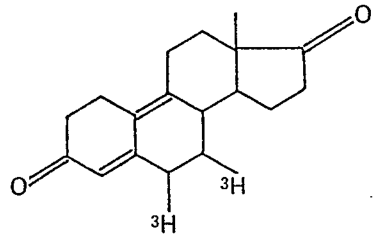

(II)

mit Hilfe eines Ketalisierungsmittels blockiert, das entstandene Produkt der allgemeinen Formel (III)

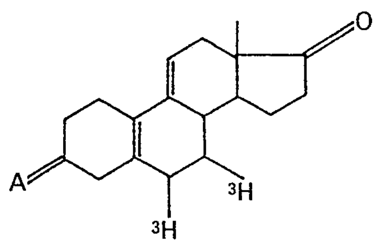

(III)

worin A eine in Form des offenen oder cyclischen ketals blockierte Oxogruppe darstellt, der Einwirkung eines Propinylierungsmittels unterzieht und das Produkt der allgemeinen Formel (IV)

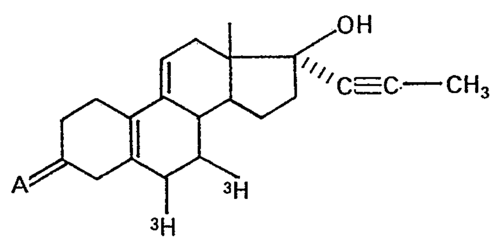

(IV)

erhält, welches man der Einwirkung eines Epoxidierungsmittels unterzieht, um das Produkt der allgemeinen Formel (V)

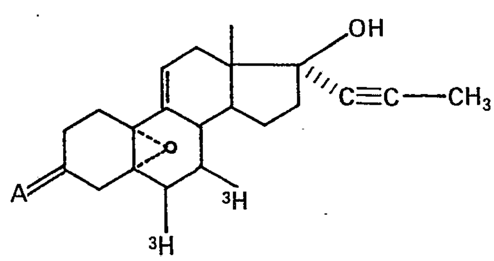

(V)

zu erhalten, mit dem man ein 4-Dimethylaminophenylmagnesiumhalogenid umsetzt, hiernach das entstandene Produkt der allgemeinen Formel (VI)

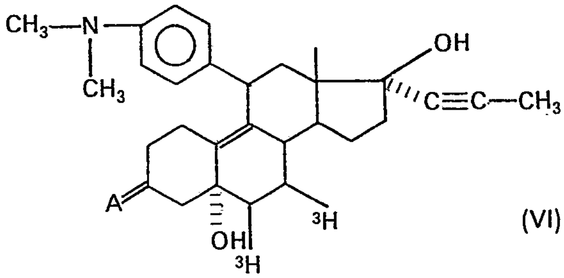

(VI)

der Hydrolyse unterzieht und das gewünschte Produkt der allgemeinen Formel (I), worin R für ein Wasserstoffatom steht, erhält, dessen Hydroxylfunktion in 17-Stellung man gegebenenfalls verethert oder verestert.

4. Verfahren gemäss Anspruch 3, dadurch gekennzeichnet, dass das Ketalisierungsmittel, mit dessen Hilfe man die Funktion in 3-Stellung blokkiert, ein Alkanol mit 1 bis 4 Kohlenstoffatomen oder ein Glykol mit 2 Kohlenstoffatomen, die gegebenenfalls durch eine oder zwei Alkylgruppen substituiert sind, wie Methyl, ist und man unter inerter Atmosphäre arbeitet, das Propinylierungsmittel, das man mit dem Produkt der allgemeinen Formel (III) umsetzt, das Propinylmagnesiumbromid ist und man unter inerter Atmosphäre arbeitet, das Epoxidierungsmittel, das man mit dem Produkt der allgemeinen Formel (IV) umsetzt, eine Persäure, wie m-Chlorperbenzoesäure, oder Wasserstoffperoxid in Anwesenheit von Hexafluoracetonhydrat ist und man unter inerter Atmosphäre arbeitet, das 4-Dimethylaminophenyl-magnesiumhalogenid, das man mit dem Produkt der allgemeinen Formel (V) umsetzt, das Bromid ist und man unter inerter Atmosphäre arbeitet, man das Produkt der allgemeinen Formel (VI) der sauren Hydrolyse mit Hilfe von Chlorwasserstoffsäure unterzieht und unter inerter Atmosphäre arbeitet.

5. Abänderung des Verfahrens gemäss Anspruch 3, dadurch gekennzeichnet, dass man die Ketonfunktion in 3-Stellung des Produkts der Formel (II) in Form eines offenen Ketals mit Hilfe eines Ketalisierungsmittels der allgemeinen Formel $R_1OH$, worin $R_1$ einen Alkylrest mit 1 bis 4 Kohlenstoffatomen bedeutet, blockiert, das entstandene Produkt der allgemeinen Formel (VII)

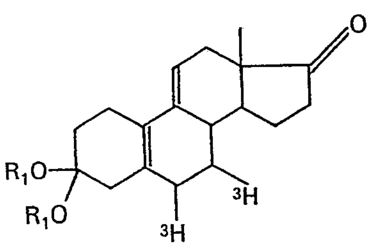

(VII)

der Einwirkung eines Propinylierungsmittels unterzieht und das Produkt der allgemeinen Formel (VIII)

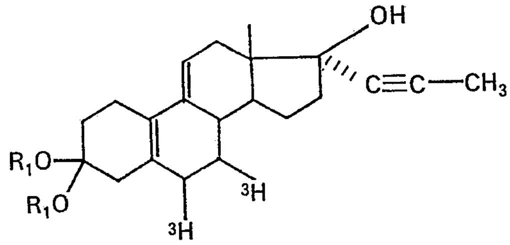

(VIII)

gewinnt, die Transketalisierung der Ketalfunktion mit Hilfe eines Glykols der Formel $R_2CHOH-CHOHR_3$ in saurem Milieu vornimmt, um das Produkt der allgemeinen Formel (IX)

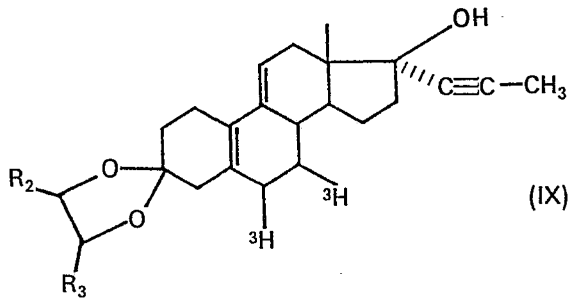

(IX)

zu erhalten, worin $R_2$ und $R_3$, die gleich oder verschieden sind, für ein Wasserstoffatom oder einen Alkylrest, wie den Methylrest, stehen, welches man nach dem vorstehend angegebenen Verfahren der Einwirkung eines Epoxidierungsmittels unterzieht, danach mit dem entstandenen Produkt ein 4-Dimethylaminophenyl-magnesiumhalogenid umsetzt und schliesslich das entstandene Produkt der Hydrolyse unterzieht, um das gewünschte Produkt der allgemeinen Formel (I) zu erhalten.

6. Verfahren gemäss Anspruch 5, dadurch gekennzeichnet, dass das ketalisierungsmittel, mit dessen Hilfe man die Ketonfunktion in 3-Stellung des Produkts der Formel (II) blockiert, Methanol ist und man unter inerter Atmosphäre arbeitet, das Propinylierungsmittel, das man mit dem Produkt der allgemeinen Formel (VII) umsetzt, das Propinylmagnesiumbromid ist und man unter inerter Atmosphäre arbeitet, das Glykol, mit dessen Hilfe man die Transketalisierung der Ketonfunktion des Produkts der allgemeinen Formel (VIII) durchführt, Ethylenglykol ist und man unter inerter Atmosphäre arbeitet.

7. Verwendung der Produkte der allgemeinen Formel (I) gemäss Anspruch 1 und insbesondere von 17β-Hydroxy-11β-(4-dimethylaminophenyl)-17α-(prop-1-inyl)-östra-4,9-dien-3-on (6,7-$^3H$) für die radio-immunologische Untersuchung und Bestimmung von nicht-radioaktivem 17β-Hydroxy-11β-(4-dimethylaminophenyl)-17α-(prop-1-inyl)-östra-4,9-dien-3-on und dessen Metaboliten in biologischen Flüssigkeiten bzw. Fluiden beim Menschen oder beim Tier.

8. Produkte der Formel (I) gemäss Anspruch 1 oder 2 zu deren Verwendung beim Menschen oder beim Tier für die radio-immunologische Untersuchung und Bestimmung von nicht-radioaktivem 17β-Hydroxy-11β-(4-dimethylamino-phe-

nyl)-17α-(prop-1-inyl)-östra-4,9-dien-3-on und dessen Metaboliten in biologischen Flüssigkeiten bzw. Fluiden.

9. Mittel für die radio-immunologische Untersuchung und Bestimmung von nicht-radioaktivem 17β-Hydroxy-11β-(4-dimethylaminophenyl)-17α-(prop-1-inyl)-östra-4,9-dien-3-on und dessen Metaboliten in biologischen Flüssigkeiten bzw. Fluiden beim Menschen oder beim Tier, enthaltend ein Produkt der allgemeinen Formel (I) gemäss Anspruch 1 und insbesondere das 17β-Hydroxy-11β-(4-di-methylaminophenyl)-17α-(prop-1-inyl)-östra-4,9-dien-3-on (6, 7-$^3$H).

10. Als neue, industrielle Produkte:

3,3-Dimethoxy-17β-hydroxy-17α-(prop-1-inyl)-östra-5(10),9(11)-dien (6,7-$^3$H),

3,3-Ethylendioxy-17β-hydroxy-17α(prop-1-inyl)-östra-5(10), 9(11)-dien (6,7-$^3$H),

3,3-Ethylendioxy-5α,10α-epoxy-17β-hydroxy-17α-(prop-1-inyl)-östra-9(11)-en (6,7-$^3$H),

3,3-Ethylendioxy-5α, 17β-dihydroxy-11β-(4-dimethyl-aminophenyl)-17α-(prop-1-inyl)-östra-9(11)-en (6,7-$^3$H).

**Patentansprüche für den Vertragsstaat: AT**

1. Verfahren zur Herstellung von Tritium-markierten, radioaktiven Östradien-Derivaten der allgemeinen Formel (I)

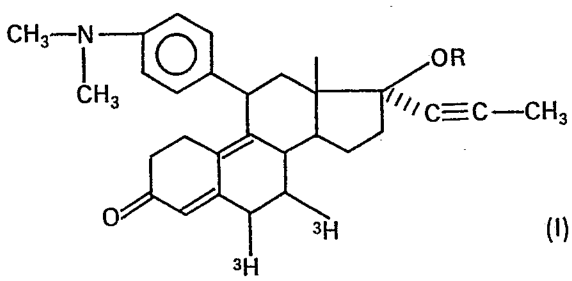

(I)

worin $^3$H für Tritium steht und R ein Wasserstoffatom, einen Alkylrest mit 1 bis 6 Kohlenstoffatomen oder einen Acylrest mit 1 bis 10 Kohlenstoffatomen darstellt, dadurch gekennzeichnet, dass man die Ketonfunktion in 3-Stellung des Produkts der Formel (II), worin das Symbol $^3$H für Tritium steht,

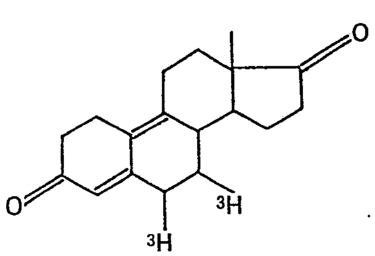

(II)

mit Hilfe eines Ketalisierungsmittels blockiert, das entstandene Produkt der allgemeinen Formel (III)

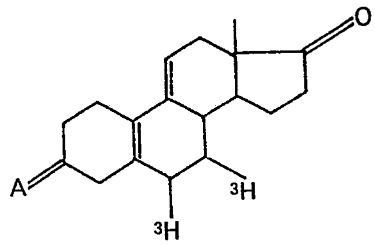

(III)

worin A eine in Form des offenen oder cyclischen Ketals blockierte Oxogruppe darstellt, der Einwirkung eines Propinylierungsmittels unterzieht und das Produkt der allgemeinen Formel (IV)

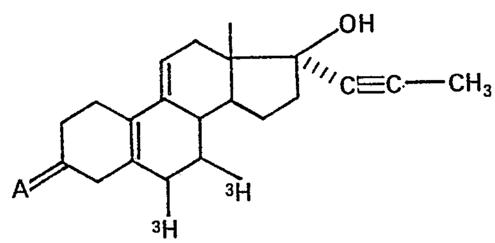

(IV)

erhält, welches man der Einwirkung eines Epoxidierungsmittels unterzieht, um das Produkt der allgemeinen Formel (V)

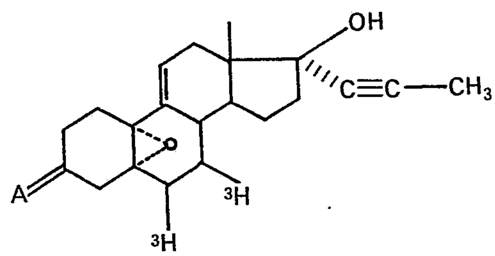

(V)

zu erhalten, mit dem man ein 4-Dimethylaminophenylmagnesiumhalogenid umsetzt, hiernach das entstandene Produkt der allgemeinen Formel (VI)

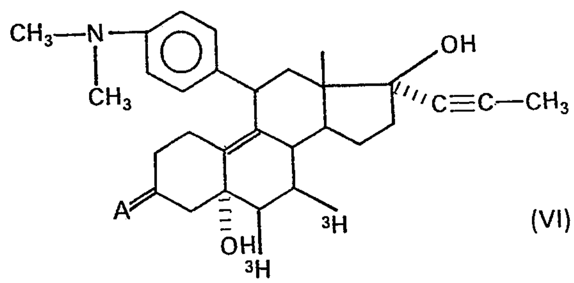

(VI)

der Hydrolyse unterzieht und das gewünschte Produkt der allgemeinen Formel (I), worin R für ein Wasserstoffatom steht, erhält, dessen Hydroxylfunktion in 17-Stellung man gegebenenfalls verethert oder verestert.

2. Verfahren gemäss Anspruch 1, dadurch gekennzeichnet, dass das Ketalisierungsmittel, mit dessen Hilfe man die Funktion in 3-Stellung blokkiert, ein Alkanol mit 1 bis 4 Kohlenstoffatomen oder ein Glykol mit 2 Kohlenstoffatomen, die gegebenenfalls mit einer oder mehreren Alkylgruppen, wie Methyl, substituiert sind, ist und man unter inerter Atmosphäre arbeitet, das Propinylierungsmittel, das man mit dem Produkt der allgemeinen Formel (III) umsetzt, das Propinylmagnesiumbromid ist und man unter inerter Atmosphäre arbeitet, das Epoxidierungsmittel, das man mit dem Produkt der allgemeinen Formel (IV) umsetzt, eine Persäure, wie m-Chlorperbenzoesäure, oder Wasserstoffperoxid in Anwesenheit von Hexafluoracetonhydrat ist und man unter inerter Atmosphäre arbeitet, das 4-Dimethylaminophenyl-magnesiumhalogenid, das man mit dem Produkt der allgemeinen Formel (V) umsetzt, das Bromid ist und man unter inerter Atmosphäre arbeitet, man das Produkt der allgemeinen Formel (VI) der sauren Hydrolyse mit Hilfe von Chlorwasserstoffsäure unterzieht und unter inerter Atmosphäre arbeitet.

3. Abänderung des Verfahrens gemäss Anspruch 1, dadurch gekennzeichnet, dass man die Ketonfunktion in 3-Stellung des Produkts der Formel (II) in Form eines offenen Ketals mit Hilfe eines Ketalisierungsmittels der allgemeinen Formel $R_1OH$, worin $R_1$ für einen Alkylrest mit 1 bis 4 Kohlenstoffatomen steht, blockiert, das entstandene Produkt der allgemeinen Formel (VII)

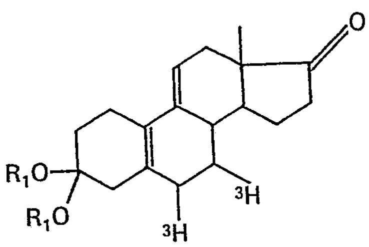

(VII)

der Einwirkung eines Propinylierungsmittels unterzieht und das Produkt der allgemeinen Formel (VIII)

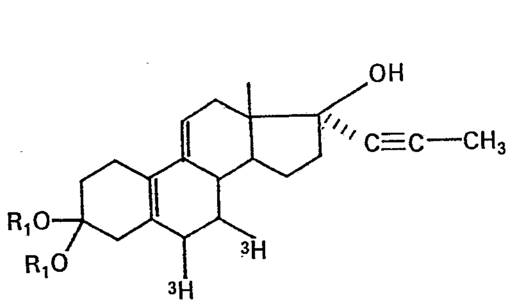

(VIII)

gewinnt, die Transketalisierung der Ketalfunktion mit Hilfe eines Glykols der Formel $R_2CHOH-CHOHR_3$ in saurem Milieu vornimmt, um das Produkt der allgemeinen Formel (IX)

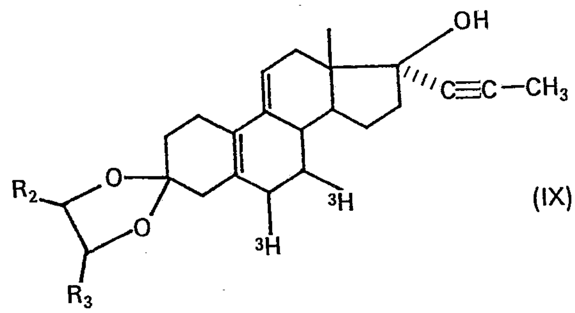

(IX)

zu erhalten, worin $R_2$ und $R_3$, die gleich oder verschieden sind, für ein Wasserstoffatom oder einen Alkylrest, wie den Methylrest, stehen, welches man nach dem vorstehend angegebenen Verfahren der Einwirkung eines Epoxidierungsmittels unterzieht, danach mit dem entstandenen Produkt ein 4-Dimethylaminophenyl-magnesiumhalogenid umsetzt und schliesslich das entstandene Produkt der Hydrolyse unterzieht, um das gewünschte Produkt der allgemeinen Formel (I) zu erhalten.

4. Verfahren gemäss Anspruch 3, dadurch gekennzeichnet, dass das Ketalisierungsmittel, mit dessen Hilfe man die Ketonfunktion in 3-Stellung des Produkts der Formel (II) blockiert, Methanol ist und man unter inerter Atmosphäre arbeitet, das Propinylierungsmittel, das man mit dem Produkt der allgemeinen Formel (VII) umsetzt, das Propinylmagnesiumbromid ist und man unter inerter Atmosphäre arbeitet, das Glykol, mit dessen Hilfe man die Transketalisierung der Ketonfunktion des Produkts der allgemeinen Formel (VIII) vornimmt, das Ethylenglykol ist und man unter inerter Atmosphäre arbeitet.

5. Verwendung der Produkte der allgemeinen Formel (I) gemäss Anspruch 1 und insbesondere von 17β-Hydroxy-11β-(4-dimethylaminophenyl)-17α(prop-1-inyl)-östra-4,9-dien-3-on (6,7-$^3$H) für die radio-immunologische Untersuchung und Bestimmung von nicht-radioaktivem 17β-Hydroxy-11β-(4-dimethylaminophenyl)-17α-(prop-1-inyl)-östra-4,9-dien-3-on und dessen Metaboliten in biologischen Flüssigkeiten bzw. Fluiden beim Menschen oder beim Tier.

6. Mittel für die radio-immunologische Untersuchung und Bestimmung von nicht-radioaktivem 17β-Hydroxy-11β-(4-dimethylaminophenyl)-17α-(prop-1-inyl)-östra-4,9-dien-3-on und dessen Metaboliten in biologischen Flüssigkeiten bzw. Fluiden beim Menschen oder beim Tier, enthaltend ein Produkt der allgemeinen Formel (I) gemäss Anspruch 1 und insbesondere 17β-Hydroxy-11β-(4-dimethyl-aminophenyl)-17α-(prop-1-inyl)-östra-4,9-dien-3-on (6,7-$^3$H).

**Claims for the contracting states BE, CH, DE, GB, IT, LI, LU, NL, SE**

1. Radioactive estradiene derivatives labelled with tritium, with the general formula (I):

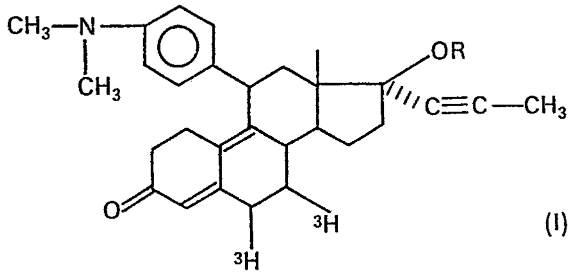

(I)

in which $^3H$ represents tritium and R represents a hydrogen atom, an alkyl radical having from 1 to 6 carbon atoms or an acyl radical having from 1 to 10 carbon atoms.

2. 17β-hydroxy-11β-(4-dimethylaminophenyl)-17-alpha-(prop-1-ynyl)-estra-4,9-dien-3-one (6,7-$^3H$).

3. Process for the preparation of the products defined in claims 1 and 2, characterised in that:
- the ketone function at position 3 of the product with the formula (II)

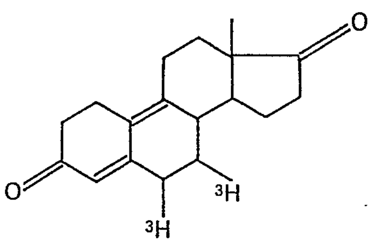

(II)

in which the symbol $^3H$ represents tritium is blocked by means of a ketalisation agent,
- the resulting product with the general formula (III)

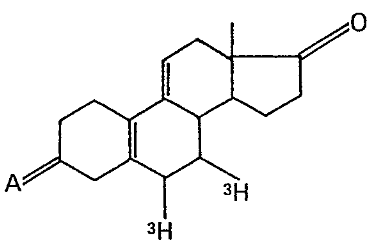

(III)

in which formula (III) A represents an oxo group blocked in the form of an open or cyclic ketal, is submitted to the action of a propynylation agent, and the product is obtained with the general formula (IV):

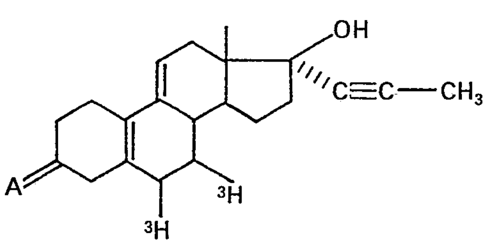

(IV)

- which is submitted to the action of an epoxidising agent in order to obtain the product with the general formula (V):

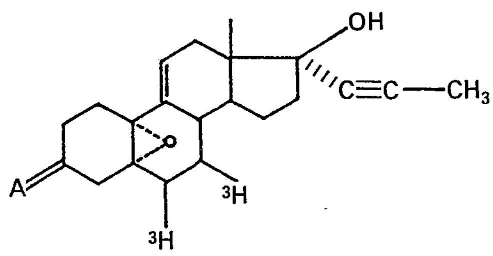

(V)

- on which a halide of 4-dimethylaminophenyl magnesium is made to act,
- the resulting product with the general formula (VI):

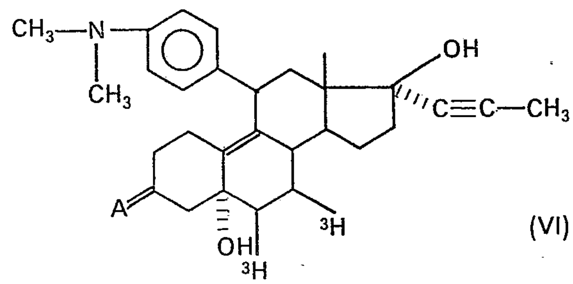

(VI)

- is submitted to hydrolysis and that the product sought with the general formula (I) is obtained, in which R represents a hydrogen atom, of which, if required, the hydroxyl function in position 17 is etherified or esterified.

4. Process according to claim 3, caracterised in that:
- the ketalising agent, by means of which the function in position 3 is blocked, is an alkanol, having from 1 to 4 carbon atoms or a glycol having 2 carbon atoms, substituted or not by one or two alkyl groups, such as methyl, and the operation is carried out under an inert atmosphere.
- the propynylating agent which is made to act on the product with the general formula (III) is propynylmagnesium bromide, and the operation is carried out under inert atmosphere.
- the epoxidising agent which is made to act on the product with the general formula (IV) is a peracid such as m-chloroperbenzoic acid or hydrogen peroxide in the presence of hexafluoroacetone hydrate, and the operation is carried out under inert atmosphere.
- the halide of 4-dimethylaminophenyl magnesium which is made to act on the product with the general formula (V) is the bromide, and the operation is carried out under inert atmosphere.
- the product of the general formula (VI) is submitted to acid hydrolysis by means of hydrochloric acid and the operation is carried out under inert atmosphere.

5. Variant of the process according to claim 3, characterised in that:
- the ketone function in position 3 of the product with the formula (II) is blocked in the form of an open ketal, by means of a ketalising agent with the general formula $R_1OH$, in which $R_1$ represents an alkyl radical having from 1 to 4 carbon atoms.

- the resulting product with the general formula (VII):

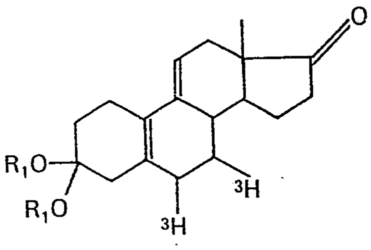

(VII)

is submitted to the action of a propynylating agent, so obtaining the product with the general formula (VIII):

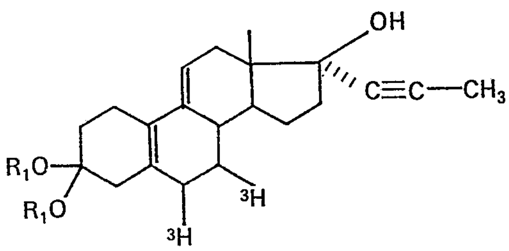

(VIII)

- the transketalisation of the ketal function is effected by means of a glycol with the formula $R_2CHOH-CHOHR_3$ in an acid medium, so as to obtain a product with the general formula (IX):

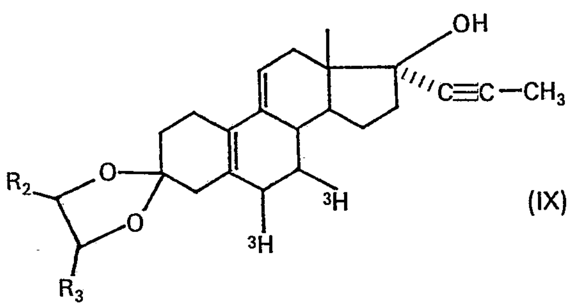

(IX)

in which $R_2$ and $R_3$, alike or different, each represents a hydrogen atom or an alkyl radical such as methyl, which, according to the process indicated above, is submitted to the action of an epoxidising agent, then a halide of 4-dimethylaminophenyl magnesium is made to act on the resulting product, which finally is submitted to hydrolysis so as to obtain the product sought with the general formula (I).

6. Process according to claim 5, characterised in that:

- the ketalisation agent by means of which the ketone function in position 3 of the product with the formula (II) is blocked is methanol and the operation is carried out under an inert atmosphere,
- the propynylating agent which is made to act on the product with the general formula (VII) is propynylmagnesium bromide, and the operation is carried out under inert atmosphere.
- the glycol by means of which the transketalisation of the ketone function of the product with the general formula (VIII) is effected is ethylene glycol, and the operation is carried out under an inert atmosphere.

7. Use of the products as defined by the general formula (I) of claim 1, and particularly of 17β-hydroxy-11β-(4-dimethylaminophenyl)-17-alpha-(prop-1-ynyl)estra-4,9-dien-3-one (6,7-$^3$H) for the study and radio-immunological determination of non-radioactive 17β-hydroxy-11β-(4-dimethylaminophenyl)-17-alpha-(prop-1-ynyl)-estra-4,9-dien-3-one and of its metabolites in biological fluids in man or animals.

8. The products with the formula (I), as defined in claim 1 or 2, for their use, in man or animals, for the study and the radio-immunological determination of non-radioactive 17β-hydroxy-11β-(4-dimethylaminophenyl)-17-alpha-(prop-1-ynyl)-estra-4,9-dien-3-one and its metabolites in biological fluids.

9. Means for the study and the radio-immunological determination of non-radioactive 17β-hydroxy-11β-(4-dimethylaminophenyl)-17-alpha-(prop-1-ynyl)-estra-4,9-dien-3-one and its metabolites in biological fluids in man or animals, comprising a product with the general formula (I) of claim 1, and in particular 17β-hydroxy-11β-(4-dimethylaminophenyl)-17-alpha-(prop-1-ynyl)-estra-4,9-dien-3-one (6,7-$^3$H).

10. As new industrial products:

- 3,3-dimethoxy-17β-hydroxy-17-alpha-(prop-1-ynyl)-estra-5(10),9(11)-diene (6,7-$^3$H),
- 3,3-ethylenedioxy-17β-hydroxy-17-alpha-(prop-1-ynyl)-estra-5(10),9(11)-diene (6,7-$^3$H),
- 3,3-ethylenedioxy-5-alpha,10-alpha-epoxy-17β-hydroxy-17-alpha-(prop-1-ynyl)-estra-9(11)-ene(6,7-$^3$H),
- 3,3-ethylenedioxy-5-alpha,17β-dihydroxy-11β-(4-dimethylamino-phenyl)-17-alpha-(prop-1-ynyl)-estra-9(11)-ene (6,7-$^3$H).

**Claims for the contracting state: AT**

1. Process for the preparation of radioactive estradiene derivatives labelled with tritium, with the general formula (I):

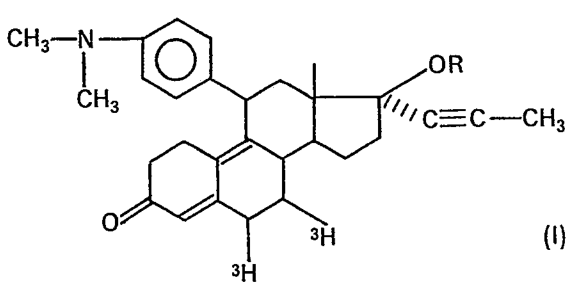

(I)

in which $^3$H represents tritium and R represents a hydrogen atom, an alkyl radical having from 1 to 6 carbon atoms or an acyl radical having from 1 to 10 carbon atoms, characterised in that:

- the ketone function at position 3 of the product with the formula (II),

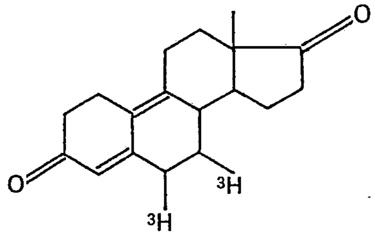

(II)

in which the symbol $^3H$ represents tritium, is blocked by means of a ketalisation agent,

- the resulting product with the general formula (III)

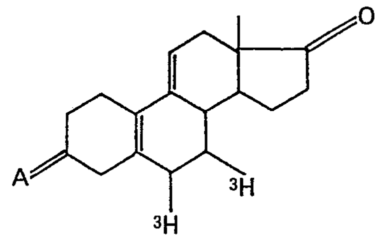

(III)

in which formula (III) A represents an oxo group blocked in the form of an open or cyclic ketal, is submitted to the action of a propynylating agent, and the product is obtained with the general formula (IV):

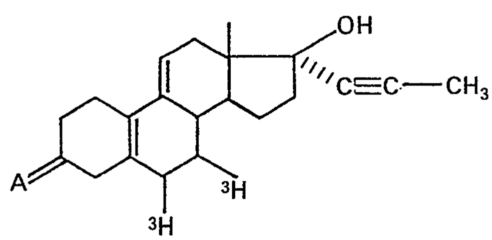

(IV)

- which is submitted to the action of an epoxidising agent in order to obtain the product with the general formula (V):

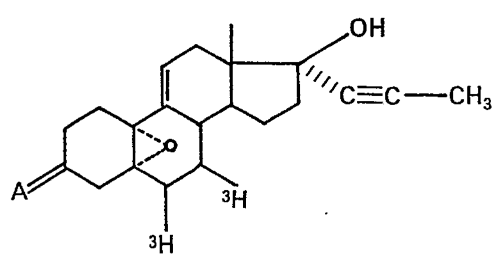

(V)

- on which a halide of 4-dimethylaminophenyl magnesium is made to act,
- the resulting product with the general formula (VI):

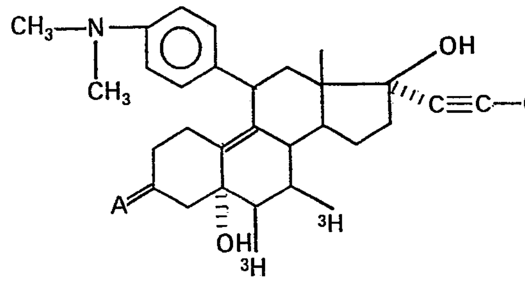

(VI)

is submitted to hydrolysis and that the product sought with the general formula (I) is obtained, in which R represents a hydrogen atom, of which, if required, the hydroxyl function in position 17 is etherified or esterified.

2. Process according to claim 1, characterised in that:

- the ketalising agent, by means of which the function in position 3 is blocked, is an alkanol, having from 1 to 4 carbon atoms or a glycol having 2 carbon atoms, substituted or not by one or two alkyl groups, such as methyl, and the operation is carried out under an inert atmosphere.
- the propynylating agent which is made to act on the product with the general formula (III) is propynylmagnesium bromide, and the operation is carried out under inert atmosphere.
- the epoxidising agent which is made to act on the product with the general fromula (IV) is a peracid such as m-chloroperbenzoic acid or hydrogen peroxide in the presence of hexafluoroacetone hydrate, and the operation is carried out under inert atmosphere.
- the halide of 4-dimethylaminophenyl magnesium which is made to act on the product with the general formula (V) is the bromide, and the operation is carried out under inert atmosphere.
- the product of the general formula (VI) is submitted to acid hydrolysis by means of hydrochloric acid and the operation is carried out under inert atmosphere.

3. Variant of the process according to claim 1, characterised in that:

- the ketone function in position 3 of the product with the formula (II) is blocked in the form of an open ketal, by means of a ketalising agent with the general formula $R_1OH$, in which $R_1$ represents an alkyl radical having from 1 to 4 carbon atoms.
- the resulting product with the general formula (VII):

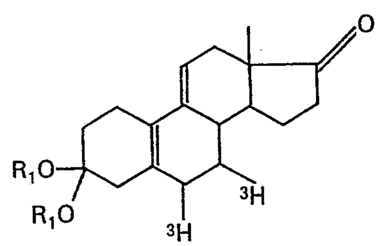

(VII)

is submitted to the action of a propynylating agent, so obtaining the product with the general formula (VIII):

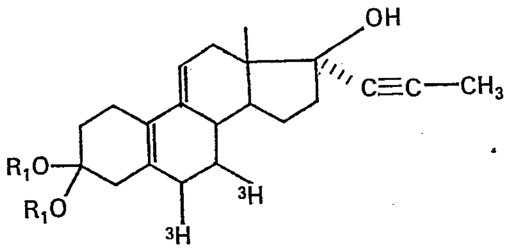

(VIII)

– the transketalisation of the ketal function is effected by means of a glycol with the formula $R_2CHOH-CHOHR_3$ in an acid medium, so as to obtain a product with the general formula (IX):

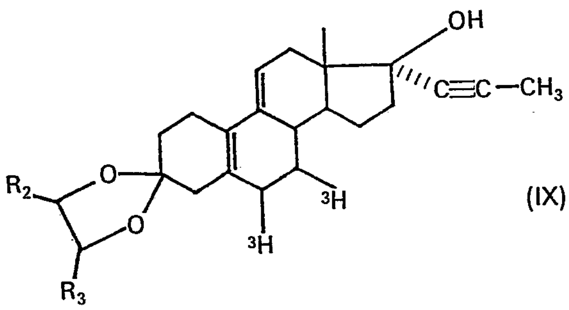

(IX)

in which $R_2$ and $R_3$, alike or different, each represents a hydrogen atom or an alkyl radical such as methyl, which, according to the process indicated above, is submitted to the action of an epoxidising agent, then a halide of 4-dimethylaminophenyl magnesium is made to act on the resulting product, which finally is submitted to hydrolysis so as to obtain the product sought with the general formula (I).

4. Process according to claim 3, characterised in that:
– the ketalisation agent by means of which the ketone function in position 3 of the product with the formula (II) is blocked is methanol and the operation is carried out under an inert atmosphere,
– the propynylating agent which is made to act on the product with the general formula (VII) is propynylmagnesium bromide, and the operation is carried out under an inert atmosphere,
– the glycol by means of which the transketalisation of the ketone function of the product with the general formula (VIII) is ethylene glycol, and the operation is carried out under an inert atmosphere.

5. Use of the products as defined by the general formula (I) of claim 1, and particularly of 17β-hydroxy-11β-(4-dimethylaminophenyl)-17-alpha-(prop-1-ynyl)estra-4,9-dien-3-one (6,7-$^3$H) for the study and radio-immunological determination of non-radioactive 17β-hydroxy--11β-(4-dimethylaminophenyl)-17-alpha-(prop-1-ynyl)-estra-4,9-dien-3-one and of its metabolites in biological fluids in man or animals.

6. Means for the study and the radio-immunological determination of non-radioactive 17β-hydroxy-11β-(4-dimethylaminophenyl)-17-alpha-(prop-1-ynyl)-estra-4,9-dien-3-one and its metabolites in biological fluids in man or animals, comprising a product with the general formula (I) of claim 1, and in particular 17β-hydroxy-11β-(4-dimethylaminophenyl)-17-alpha-(prop-1-ynyl)-estra-4,9-dien-3-one (6,7-$^3$H).